# Europäisches Patentamt

## European Patent Office

## Office européen des brevets

(19)

(11) Veröffentlichungsnummer: **0 006 190**
**B1**

(12)

# EUROPÄISCHE PATENTSCHRIFT

(45) Veröffentlichungstag der Patentschrift:
01.07.81

(51) Int. Cl.³: **C 07 C 113/04**

(21) Anmeldenummer: 79101804.7

(22) Anmeldetag: 07.06.79

(54) **Verfahren zur kontinuierlichen Diazotierung von Aminen.**

(30) Priorität: 12.06.78 DE 2825655

(43) Veröffentlichungstag der Anmeldung:
09.01.80 Patentblatt 80/1

(45) Bekanntmachung des Hinweises auf die Patenterteilung:
01.07.81 Patentblatt 81/26

(84) Benannte Vertragsstaaten:
**BE CH DE FR GB IT**

(56) Entgegenhaltungen:
**DE-A-2 635 778**
**DE-A-960 205**
**FR-A-2 309 517**

(73) Patentinhaber: **HOECHST AKTIENGESELLSCHAFT,
Postfach 80 03 20, D-6230 Frankfurt/Main 80 (DE)**

(72) Erfinder: **Behringer, Hartmut, Dr., Rotdornweg 6,
D-5042 Erftstadt (DE)**
Erfinder: **Karrenbauer, Kurt, Dr., Lange Heide 18,
D-5042 Erftstadt (DE)**

## Verfahren zur kontinuierlichen Diazotierung von Aminen

Der Diazotierung von primären aromatischen Aminen unter Bildung von Diazoniumsalzen kommt besonders im Hinblick auf die Herstellung von Azofarbstoffen eine erhebliche wirtschaftliche Bedeutung zu.

Üblicherweise erfolgt die Diazotierung von primären aromatischen Aminen in diskontinuierlicher Verfahrensweise, wobei in einem Rührbehälter zunächst das Amin in wässeriger Mineralsäure, wie Salz- oder Schwefelsäure, gelöst oder suspendiert wird. Allgemein wird hierbei ein Überschuß von 2,5 bis 3 Äquivalenten Mineralsäure pro Äquivalent Amin verwendet. Anschließend wird in die Lösung oder Suspension eine wässerige konzentrierte Natriumnitritlösung bis zum Erreichen des Äquivalenzpunktes eingetragen. Durch Eiszugabe wird die Temperatur des Reaktionsgemisches auf etwa Null bis 5°C gehalten. Das dabei gebildete Diazoniumsalz bzw. dessen wässerige Lösung wird in der Regel nach Filtration für Kupplungsreaktion zur Bildung von Azofarbstoffen verwendet.

Diese bisher großtechnisch angewandte Verfahrensweise weist jedoch erhebliche Mängel auf. So ist beispielsewise die Raum-Zeit-Ausbeute unbefriedigend. Da der für die Diazotierung bestimmte Rührbehälter sowohl für die Vorbereitung der wässerigen mineralsauren Lösung oder Suspension des Amins als auch für die Durchführung der Diazotierungsreaktion und anschließende Lagerung der Diazoniumsalzlösung bis zu deren Weiterverarbeitung verwendet wird, ist der Behälter zeitweise für die Durchführung weiterer Diazotierungsreaktionen blockiert, was von Nachteil ist. Ein weiterer Nachteil der diskontinuierlichen Diazotierung stellt die relativ lange Verweilzeit des einmal gebildeten Diazoniumsalzes im Rührbehälter bis zur Vollendung der Gesamtreaktion dar. Bekanntlich sind Diazoniumsalze mehr oder weniger leicht zersetzlich, wobei die Zersetzungsprodukte einen erheblichen Einfluß auf die Qualität von Folgeprodukten ausüben, so daß es wichtig ist, die Verweil- bzw. Lagerzeiten von Diazoniumsalzlösungen bis zu deren Weiterverarbeitung grundsätzlich kurz und konstant zu halten oder zumindest ohne Blockierung des Diazotierbehälters variieren zu können.

Ein kontinuierliches Verfahren zur Diazotierung von Aminen wird in der deutschen Patentschrift Nr. 960 205 beschrieben. Das Verfahren ist dadurch gekennzeichnet, daß man in ein vorgelegtes, vorgekühltes Lösungs- oder Verdünnungsmittel, das sich im Laufe des Verfahrens mit Reaktionsmischung anreichert, gleichzeitig und kontinuierlich ein diazotierbares Amin als solches oder als Salz, gegebenenfalls in Mischung mit geeigneten Lösungsmitteln, dazu ein Diazotiermittel sowie gegebenenfalls eine oder mehrere Säuren sowie Verdünnungsmittel einführt, wobei man die Zugabe der Reaktionsteilnehmer nach der Zusammensetzung des Verfahrensproduktes reguliert und das Verfahrensprodukt kontinuierlich abzieht.

Das bekannte Verfahren besitzt den Nachteil, daß die Diazoniumsalzlösung, im Gegensatz zur diskontinuierlichen Arbeitsweise, während der gesamten Reaktionsdauer einen Überschuß an salpetriger Säure aufweist, wodurch die Zersetzung des Diazoniumsalzes begünstigt und die Qualität von Folgeprodukten des Diazoniumsalzes verschlechtert wird. Schließlich stellt die Maßnahme der ständigen Kreislaufführung der Hauptmenge der Diazoniumsalzlösung aufgrund der damit verbundenen Zersetzungsgefahr des Diazoniumsalzes einen Nachteil des bekannten Verfahrens dar. Die Nachteile der bekannten Verfahren werden durch vorliegende Erfindung, welche die kontinuierliche Diazotierung von diazotierbaren Aminen in guter Ausbeute ermöglicht, überwunden.

Gegenstand der Erfindung ist ein Verfahren zur kontinuierlichen Diazotierung von primären aromatischen Aminen durch Umsetzung einer wässerigen, mineralsauren Lösung oder Suspension des Amins mit einer wässerigen Natriumnitritlösung, welches dadurch gekennzeichnet ist, daß man kontinuierlich in den unteren Teil eines vertikalen zylindrischen Diazotierbehälters eine wässerige, mineralsaure Lösung oder Suspension eines diazotierbaren, primären aromatischen Amins und gleichzeitig über eine oder mehrere, seitlich am Behälter übereinander angeordnete Zuführungsstellen eine wässerige Natriumnitritlösung, wobei Amin und Nitrit in stöchiometrischem Verhältnis oder das Nitrit in unterstöchiometrischer Menge und die Säure in der mineralsauren Lösung im Überschuß von 1 bis 3 Äquivalenten pro Äquivalent Amin vorliegen, einleitet und unter Rühren des Gemiches und Erzeugung eines laminaren Flüssigkeitsstromes bei einer Temperatur von etwa 5 bis 30°C umsetzt, wonach man das Reaktionsgemisch im oberen Teil des Diazotierbehälters in Abhängigkeit von der Diazotierungsgeschwindigkeit des Amins an einer Stelle abzieht, an der das Reaktionsgemisch nur einen geringen Überschuß an salpetriger Säure aufweist, wobei diese Stelle um so höher liegt, je geringer die Diazotierungsgeschwindigkeit des eingesetzten Amins ist, und daß man das abgezogene Reaktionsgemisch filtriert und das diazoniumsalzhaltige Filtrat einem Verweilbehälter zuführt.

Das erfindungsgemäße Verfahren läßt in Abhängigkeit von der Diazotiergeschwindigkeit des eingesetzten aromatischen Amins verschiedene Ausführungsformen zu. So hat es sich als zweckmäßig erwiesen, beim Einsatz von leicht diazotierbaren Aminen mit hoher Diazotiergeschwindigkeit die wässerige Natriumnitritlösung ausschließlich über die unterste Zuführungsstelle in den Diazotierbehälter einzuleiten.

Besteht jedoch die Aufgabe, schwer diazotierbare Amine mit verminderter Diazotiergeschwindigkeit zu diazotieren, so ist es vorteilhaft, die wässerige Natriumnitritlösung gleichzeitig über mehrere Zuführungsstellen in den Diazotierbehälter einzuleiten, wobei die an den einzelnen Zuführungsstellen in den Behälter eingeleiteten Mengen an Natriumnitritlösung von unten nach oben in dem Maße abnehmen, daß das Reaktionsgemisch nur im Bereich der obersten Zuführungsstelle einen geringen Überschuß an salpetriger Säure aufweist. Eine andere Ausführungsform sieht in diesem Falle auch vor, die wässerige Nitritlösung in einer unterstöchiometrischen Menge gegenüber dem Amin in den Diazotierbehälter einzuleiten, nicht umgesetztes Amin von dem abgezogenen Reaktionsgemisch abzufiltrieren und den Filterrückstand erneut der Diazotierung zuzuführen.

Ein weiteres bevorzugtes Merkmal der Erfindung besteht darin, die Diazotierung des Amins unabhängig von der Art des Amins bei einer Temperatur von 10−20°C durchzuführen. Für den einwandfreien Ablauf des Verfahrens der Erfindung ist es wichtig, daß das Reaktionsgemisch an der Entnahmestelle des Diazotierbehälters einen minimalen, durch Tüpfelreaktion auf Kaliumjodidstärkepapier erkennbaren Gehalt an salpetriger Säure aufweist. Letztgenannter Überschuß ist ein Indiz für die Vollständigkeit der Reaktion. Eine Regulierung der Diazotierung ist erfindungsgemäß dadurch gegeben, daß man nichtumgesetztes Amin im diazoniumsalzhaltigen Filtrat auch außerhalb des Diazotierbehälters im anschließenden Verweilbehälter durch Zugabe von wässeriger Natriumnitritlösung nachdiazotieren kann.

Im allgemeinen beträgt die Verweilzeit für Diazoniumsalzlösungen, welche aus leicht diazotierbaren Aminen hergestellt wurden, etwa 1 bis 10 Minuten und für solche aus schwer diazotierbaren Aminen etwa 10 bis 30 Minuten.

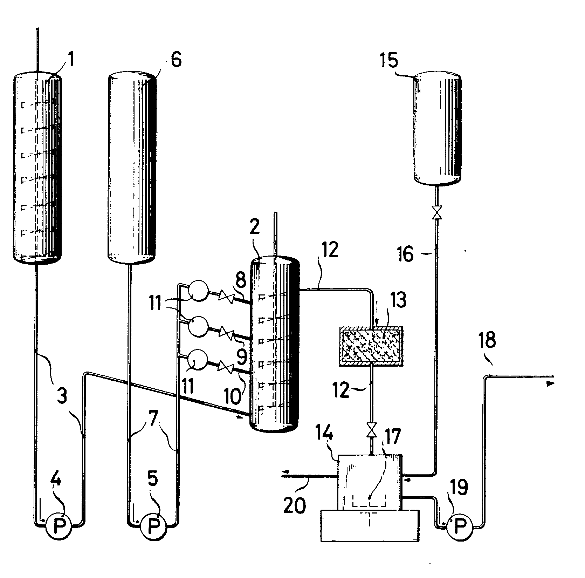

Das Verfahren der Erfindung wird nachstehend anhand der beigefügten Zeichnung näher erläutert:

Eine wässerige Lösung oder Suspension aus primären, aromatischem Amin und Mineralsäure, die sich in dem mit Rührer ausgestatteten Behälter 1 befindet, wird in den ebenfalls mit Rührer versehenen Diazotierbehälter 2 über die Leitung 3 mit Hilfe der Dosierpumpe 4 gefördert. Gleichzeitig wird durch die Pumpe 5 von dem Vorratsbehälter 6 in den Diazotierbehälter 2 über die Leitung 7 sowie über die Zuführungsstellen 8, 9 und 10 eine berechnete Menge einer wässerigen Natriumnitritlösung gepumpt, wobei die Mengenmessung durch die Rotameter 11 erfolgt. Zur Kühlung des Reaktionsgemisches ist der Diazotierbehälter 2 mit einem nichtgezeichneten Kühlmantel umgeben. Die Rührarme des Rührers im Diazotierbehälter 2 sind so gestellt, daß sie eine gute Rührwirkung in der Ebene senkrecht zur Rührachse bewirken, wobei die Rührgeschwindigkeit zur Erzeugung eines laminaren Flüssigkeitsstromes in Richtung der Rührachse bemessen ist. Aufgrund der laminaren Strömungsverhältnisse im Diazotierbehälter 2 wird die Einstellung einer definierten Verweilzeit des Diazoniumsalzes im Behälter ermöglicht, wobei die Verweilzeit ausschließlich von der Größe des Diazotierbehälters 2 und der in der Zeiteinheit zugeführten Flüssigkeitsmenge abhängig ist.

Das über Leitung 12 abströmende Reaktionsgemisch wird in der Filtervorrichtung 13 filtriert und im Verweilbehälter 14 gesammelt.

Eine gegebenenfalls erforderliche Nachdiazotierung der im Verweilbehälter 14 gesammelten wässerigen Diazoniumsalzlösung zur Umsetzung von in der Diazoniumsalzlösung gelöstem nichtumgesetztem Amin kann durch Zuführung von wässeriger Natriumnitritlösung aus dem Behälter 15 über die Leitung 16 in den Verweilbehälter 14 erfolgen. Zur guten Durchmischung des Inhaltes des Verweilbehälters 14 ist der Magnetrührer 17 bestimmt. Über Leitung 18 erfolgt mit Hilfe der Pumpe 19 die Entnahme der Diazoniumsalzlösung. Über die Leitung 20 kann gegebenenfalls an das Filter 13 Vakuum angelegt werden.

Das Verfahren der Erfindung besitzt gegenüber den einschlägigen bekannten Verfahren verschiedene Vorteile. Ein Vorteil besteht darin, daß die Diazotierung der Amine aufgrund der kurzen Verweilzeit des Reaktionsgemisches im Diazotierbehälter mit einfacher Wasserkühlung und somit unter Verzicht auf die sonst übliche Eiskühlung durchgeführt werden kann. Ein weiterer Vorteil ergibt sich bei der Diazotierung von schwer diazotierbaren Aminen durch Anwendung eines stöchiometrischen Nitritunterschusses und Rückführung des in der Filtervorrichtung abgetrennten nicht umgesetzten Amins in den Diazotierprozesses. Diese Arbeitsweise ermöglicht eine kurze Verweilzeit des Diazoniumsalzes im Reaktor sowie eine lediglich durch Regulierung der Nitritmenge einfache Steuerung des Verfahrensablaufes.

Schließlich ergibt sich in der Nachschaltung eines Verweilbehälters im Anschluß an den Diazotierbehälter der Vorteil, die Verweilzeit der Diazoniumsalzlösung regulieren und damit die Qualität der Diazoniumsalzlösung bzw. der daraus hergestellten Azofarbstoffe gezielt und reproduzierbar beeinflussen zu können.

### Beispiel 1

253 g (1 Mol) 3,3'-Dichlorbenzidin wurden im Behälter (1) in 2500 ml Wasser suspendiert und die Suspension 15 Minuten gerührt. Danach wurden 510 ml 31gewichtsprozentige Salzsäure zugegeben und die Mischung weitere 15 Minuten gerührt. Die erhaltene Suspension wurde mittels der Dosierpumpe (4) innerhalb von einer Stunde kontinuierlich in den unteren Teil des zylindrischen Diazotierbehälters (2) gepumpt, der einen Durchmesser von 4 cm und eine Höhe von 25 cm besaß. Gleichzeitig wurde 20gewichtsprozentige wässerige Natriumnitritlösung vom Behälter (6) mittels der Dosierpumpe (5) über die Zuführungsstelle (10) in den Diazotierbehälter (2) kontinuierlich in solcher Menge zugegeben, daß die aus dem Diazotierreaktor abfließende Diazoniumsalzlösung einen schwachen Nitritüberschuß enthielt. Der Verbrauch an Natriumnitritlösung betrug 600 ml oder 2 Mol. Die Temperatur im Diazotierbehälter wurde mittels Kühlwasser bei etwa 15°C gehalten. Die erhaltene Diazoniumsalzlösung war nach Filtration hellgelb und klar. Der Umsatz war 100%ig.

### Beispiel 2

304 g (2 Mol) 3-Nitro-4-aminotoluol wurden mit 850 ml Wasser und 500 ml 31gewichtsprozentiger Salzsäure 15 Minuten im Behälter (1) gerührt. Die erhaltene Suspension wurde mittels der Dosierpumpe (4) innerhalb von 1 Stunde kontinuierlich in den unteren Teil des Diazotierbehälters (2) gepumpt. Gleichzeitig wurde 20gewichtsprozentige Natriumnitritlösung über die Zuführungsstellen (8, 9 und 10) in den Diazotierbehälter (2) kontinuierlich in solchen Mengen zugegeben, daß im Bereich der Zuführungsstellen (9 und 10) im Behälter (2) kein Nitritüberschuß vorhanden war und im Bereich der Zuführungsstelle (8) ein schwacher Nitritüberschuß nachweisbar war. Die Hauptmenge der Nitritlösung wurde dabei unten, eine kleinere Menge in der Mitte und nur eine geringe Menge oben in den Behälter (2) eingeleitet. Die Temperatur im Behälter (2) wurde mittels Kühlwasser bei ca. 20°C gehalten. Die hinter dem Filter abfließende Diazoniumsalzlösung war rötlich bis hellviolett gefärbt und klar. Der Umsatz war 100%ig.

### Beispiel 3

Es wurde analog Beispiel 2 verfahren, wobei jedoch die Temperatur des Diazotierbehälters (2) lediglich 15°C betrug. Außerdem wurden nur 90 Gew.-% der in Beispiel 2 eingesetzten Menge an Natriumnitritlösung verwendet, wobei die Lösung ausschließlich über die unterste Zuführungsstelle (10) in den Diazotierbehälter eingeleitet wurde. Das überschüssige 3-Nitro-3-aminotoluol wurde in der Filtervorrichtung (13) vom Reaktionsgemisch abgetrennt, in den Behälter (1) zurückgeführt und für den folgenden Ansatz verwendet, der sich dann zu 90% aus frischem und zu 10% aus rückgewonnenem 3-Nitro-4-aminotoluol zusammensetzte. Die Kreislaufführung des zurückgewonnenen Amins wurde neunmal ohne Beeinträchtigung der Qualität des Diazoniumsalzes durchgeführt. Das im 10. Durchgang als Filterrückstand erhaltene Amin wurde zur Ausschleusung der Verunreinigungen verworfen. Die als Filtrat erhaltene Diazoniumsalzlösung war durch gelöstes 3-Nitro-4-aminotoluol braun gefärbt. Die Lösung wurde deshalb im Verweilbehälter (14) durch Zugabe einer geringen Menge Natriumnitritlösung nachdiazotiert. Die Farbe der Diazoniumsalzlösung schlug dabei in rötlichviolett um. Nach einer Verweilzeit von 15 Minuten im Behälter (14) wurde die Diazoniumsalzlösung abgezogen und weiterverarbeitet.

### Beispiel 4

Es wurde analog Beispiel 3 verfahren, wobei jedoch einmal die Verweilzeit der Diazoniumsalzlösung im Behälter (14) auf 5 Minuten vermindert und zum anderen auf 30 Minuten erhöht wurde. Aufgrund der unterschiedlichen Verweilzeiten ergab sich eine Qualitätsveränderung des Diazoniumsalzes, welche sich im Farbton des jeweils aus dem Diazoniumsalz hergestellten Azofarbstoffes äußerte.

### Beispiel 5

345 g (2 Mol) 4-Chlor-2-nitranilin wurden mit 136 ml Wasser zu einer Paste angerührt und danach 520 ml 31gewichtsprozentige Salzsäure zugegeben. Die Mischung wurde 15 Minuten gerührt und anschließend mit weiteren 662 ml Wasser und 8 ml einer 10gewichtsprozentigen Lösung von dibutyliertem Naphthalinsulfonat (Leonil DB®, Hoechst Aktiengesellschaft, Frankfurt/M) versetzt. Die so erhaltene Suspension wurde in den unteren Teil des Diazotierbehälters (2) kontinuierlich innerhalb 1 Stunde eingeleitet. Der Diazotierbehälter (2) besaß einen Durchmesser von 4 cm und eine Höhe von 70 cm. Gleichzeitig wurde Natriumnitritlösung über die Zuführungsstellen 8, 9 und 10 in den Behälter (2) eingeleitet. Die Temperatur im Behälter (2) wurde mittels Solekühlung bei 10°C gehalten. Der Umsatz betrug unter diesen Bedingungen 93%. Das nichtumgesetzte 4-Chlor-2-nitranilin wurde, wie in Beispiel 3 beschrieben, im Kreislauf geführt. Die aus dem Filter abfließende Diazoniumsalzlösung wurde zur Umsetzung des noch gelösten Amins im Behälter (14) mit einer kleinen Menge Natriumnitritlösung nachdiazotiert.

### Patentansprüche

1. Verfahren zur kontinuierlichen Diazotierung von primären aromatischen Aminen durch Umsetzung einer wässerigen, mineralsauren Lösung oder Suspension des Amins mit einer wässerigen Natriumnitritlösung, dadurch gekennzeichnet, daß man kontinuierlich in den unteren Teil eines vertikalen zylindrischen Diazotierbehälters eine wässerige, mineralsaure Lösung oder Suspension eines diazotierbaren, primären aromatischen Amins und gleichzeitig über eine oder mehrere, seitlich am Behälter übereinander angeordnete Zuführungsstellen eine wässerige Natriumnitritlösung, wobei die Säure in der mineralsauren Lösung im Überschuß von 1 bis 3 Äquivalenten pro Äquivalent Amin und Amin und Nitrit in stöchiometrischem Verhältnis oder das Nitrit in unterstöchiometrischer Menge vorliegen, einleitet und unter Rühren des Gemisches und Erzeugung eines laminaren Flüssigkeitsstromes bei einer Temperatur von etwa 5 bis 30°C umsetzt; wonach man das Reaktionsgemisch im oberen Teil des Diazotierbehälters in Abhängigkeit von der Diazotierungsgeschwindigkeit des Amins an einer Stelle abzieht, an der das Reaktionsgemisch nur einen geringen Überschuß an salpetriger Säure aufweist, wobei diese Stelle um so höher liegt, je geringer die Diazotierungsgeschwindigkeit des eingesetzten Amins ist, und daß man das abgezogene Reaktionsgemisch filtriert und das diazoniumsalzhaltige Filtrat einem Verweilbehälter zuführt.

2. Verfahren nach Anspruch 1, dadurch gekennzeichnet, daß man beim Einsatz leicht diazotierbarer Amine mit hoher Diazotiergeschwindigkeit die wässerige Natriumnitritlösung ausschließlich über die unterste Zuführungsstelle in den Diazotierbehälter einleitet.

3. Verfahren nach Anspruch 1, dadurch gekennzeichnet, daß man beim Einsatz schwer diazotierbarer Amine mit verminderter Diazotiergeschwindigkeit die wässerige Natriumnitritlösung gleichzeitig über mehrere Zuführungsstellen in den Diazotierbehälter einleitet, wobei die an den einzelnen Zuführungsstellen in den Behälter eingeleiteten Mengen an Natriumnitritlösung von unten nach oben in dem Maße abnehmen, daß das Reaktionsgemisch im Bereich der obersten Zuführungsstelle nur einen geringen Überschuß an salpetriger Säure aufweist.

4. Verfahren nach Anspruch 1, dadurch gekennzeichnet, daß man beim Einsatz schwer diazotierbarer Amine mit verminderter Diazotiergeschwindigkeit die wässerige Natriumnitritlösung in einer unterstöchiometrischen Menge gegenüber dem Amin in den Diazotierbehälter einleitet, nicht umgesetztes Amin von dem abgezogenen Reaktoinsgemisch abfiltriert und den Filterrückstand erneut der Diazotierung zuführt.

5. Verfahren nach einem der Ansprüche 1—4, dadurch gekennzeichnet, daß man die Diazotierung bei einer Temperatur von 10—20°C durchführt.

6. Verfahren nach einem der Ansprüche 1—5, dadurch gekennzeichnet, daß das Reaktionsgemisch an der Entnahmestelle des Diazotierbehälters einen minimalen, durch Tüpfelreaktion auf Kaliumjodidstärkepapier erkennbaren Gehalt an salpetriger Säure aufweist.

7. Verfahren nach einem der Ansprüche 1—6, dadurch gekennzeichnet, daß man nichtumgesetztes Amin im diazoniumsalzhaltigen Filtrat im Verweilbehälter durch Zugabe von wässeriger Natriumnitritlösung nachdiazotiert.

## Claims

1. Process for the continuous diazotization of primary aromatic amines by reacting an aqueous solution or suspension of the amine in a mineral acid with an aqueous sodium nitrite solution, which comprises: supplying continuously the lower portion of a cylindrical diazotization vessel placed in upright position with an aqueous mineral acid solution or suspension of a diazotizable primary aromatic amine and supplying the vessel simultaneously, via one or more inlets arranged one above the other so as to open laterally thereinto, with an aqueous sodium nitrite solution, the amine and nitrite being used in stoichiometric proportions or the nitrite being used in a stiochiometric deficiency and the acid being used in an excess of about 1 to 3 equivalents per amine equivalent in the mineral acid solution; reacting the resulting mixture with agitation and while producing a laminar flow of liquid matter at temperatures of about 5 to 30°C; removing reaction mixture, in accordance with the diazotization velocity of the particular amine used, from the upper portion of the diazotization vessel at a place where the reaction mixture just contains a slight excess of nitrous acid, said place being situated in said vessel at a level which is the higher the lower the diazotization velocity of the amine used; filtering the reaction mixture removed and delivering diazonium salt-containing solution to a sojourn vessel.

2. Process as claimed in claim 1, wherein in the event of readily diazotizable amines of high diazotization velocity being used, the aqueous sodium nitrite solution is introduced into the diazotization vessel exclusively through the lowermost inlet opening thereinto.

3. Process as claimed in claim 1, wherein in the event of difficultly diazotizable amines of reduced diazotization velocity being used, the aqueous sodium nitrite solution is introduced simultaneously through a plurality of inlet opening into the diazotization vessel, the sodium nitrite solution being admitted through the individual inlets in quantities decreasing from below to above so as to have a minor excess proportion of nitrous acid in the reaction mixture just within the region of the uppermost inlet.

4. Process as claimed in claim 1, wherein in the event of difficultly diazotizable amines of reduced diazotization velocity being used, a stoichiometric deficiency, with respect to the amine, of aqueous sodium nitrite solution is introduced into the diazotization vessel; unreacted amine is filtered from the reaction mixture removed and the filter residue is recycled to the diazotization vessel.

5. Process as claimed in any of claims 1 to 4, wherein the diazotization is effected at a temperature of 10 to 20°C.

6. Process as claimed in any of claims 1 to 5, wherein the reaction mixture contains, at the discharge place from the diazotization vessel, some minor excess proportion of nitrous acid recognizable by spot reaction on potassium iodide starch paper.

7. Process as claimed in any of claims 1 to 6, wherein unreacted amine present in the diazonium salt-containing filtrate is post-diazotized in the sojourn vessel by addition of aqueous sodium nitrite solution.

## Revendications

1. Procédé de diazotation en continu d'amines aromatiques primaires par réaction d'une solution ou suspension aqueuse de l'amine dans un acide minéral avec une solution aqueuse de nitrite de sodium, caractérisé en ce que l'on introduit en continu dans la partie inférieure d'un récipient de diazotation cylindrique vertical la solution ou suspension aqueuse de l'amine aromatique primaire diazotable dans un acide minéral et, simultanément, en un ou plusieurs points d'admission latéraux disposés l'un audes-sus de l'autre, une solution aqueuse de nitrite de sodium, l'amine et le nitrite étant utilisés en proportions stoechiométriques ou le nitrite étant utilisé en proportion inférieure à la proportion stoechiométrique et l'acide étant utilisé en excès de 1 à 3 équivalents par équivalent d'amine dans la solution d'acide minéral, et on fait réagir en agitant le mélange et en établissant un écoulement laminaire du liquide à des températures comprises entre environ 5 et 30°C, on soutire ensuite le mélange de réaction à la partie supérieure du récipient de diazotation en fonction de la vitesse de diazotation de l'amine en un point où le mélange de réaction ne contient qu'un faible excès d'acide nitreux, ce point de situant à un niveau d'autant plus élevé que la vitesse de diazotation est plus faible, on filtre le mélange de réaction soutiré et on amène le filtrat contenant le sel de diazonium à un récipient de stockage.

2. Procédé selon la revendication 1, caractérisé en ce que, dans le cas où il s'agit d'amines facilement diazotables à grande vitesse de diazotation, on introduit la solution aqueuse de nitrite de sodium dans le récipient de diazotation uniquement au point le plus bas.

3. Procédé selon la revendication 1, caractérisé en ce que, dans le cas où il s'agit d'amines difficilement diazotables à vitesse de diazotation réduite, on introduit la solution aqueuse de nitrite de sodium en plusieurs points dans le récipient de diazotation, les quantités de solution de nitrite de sodium introduites par les points d'admission individuels diminuant de bas en haut dans la mesure où le mélange réactionnel ne présente un faible excès d'acide nitreux que dans la région du point d'admission le plus haut.

4. Procédé selon la revendication 1, caractérisé en ce que, dans le cas où il s'agit d'amines difficilement diazotables à vitesse de diazotation réduite, on introduit dans le récipient de diazotation moins que la quantité stoechiométrique, par rapport à l'amine, de la solution aqueuse de nitrite, on soumet le mélange de réaction soutiré à une filtration pour le débarrasser de l'amine n'ayant pas réagi et on soumet le résidu de filtration à une nouvelle diazotation.

5. Procédé selon l'une quelconque des revendications 1 à 4, caractérisé en ce que l'on diazote l'amine à une température comprise entre 10 et 20°C.

6. Procédé selon l'une quelconque des revendications 1 à 5, caractérisé en ce que le mélange de réaction présente au point de sortie une teneur minimale en acide nitreux décelable par réaction par touches sur papier à l'amidon-iodure de potassium.

7. Procédé selon l'une quelconque des revendications 1 à 6, caractérisé en ce que l'on soumet l'amine n'ayant pas réagi présente dans le filtrat contenant le sel de diazonium à une post-diazotation dans le récipient de stockage par addition d'une solution aqueuse de nitrite de sodium.